Europäisches Patentamt

European Patent Office  (11) Numéro de publication: **0 113 626**

Office européen des brevets  **B1**

(12) **FASCICULE DE BREVET EUROPEEN**

(45) Date de publication du fascicule du brevet: 26.10.88  (51) Int. Cl.⁴: **C 12 N 9/96, A 23 K 1/165**

(21) Numéro de dépôt: 83402461.4

(22) Date de dépôt: 19.12.83

| E R R A T U M |

(SEITE, SPALTE, ZEILE)
(PAGE, COLUMN, LINE)
(PAGE, COLONNE, LIGNE)

DIE TEXTSTELLE :
TEXT PUBLISHED :
LE PASSAGE SUIVANT :

LAUTET BERICHTIGT:
SHOULD READ :
DEVRAIT ETRE LU :

| Texte publié | Page | Ligne | Devrait être lu |
|---|---|---|---|
| 2. Procédé selon l'une quelconque des revendications 1 et 2, | 6 | 3 | 2. Procédé selon la revendication 1, |
| des revendications 1 à 3, | 6 | 5 | des revendications 1 ou 2, |
| des revendications 1 à 4, | 6 | 7 | des revendications 1 à 3, |
| selon la revendication 5, | 6 | 9 | selon la revendication 4, |
| selon la revendication 5, | 6 | 11 | selon la revendication 4, |
| des revendications 1 à 7. | 6 | 13 | des revendications 1 à 6. |
| revendications 1 à 7. | 6 | 16 | revendications 1 à 6. |
| weight. | 6 | 58 | weight of at least one liquid enzyme stabilised by the process according to any one of claims 1 to 6. |

Tag der Entscheidung über die Berichtigung )
Date of decision on rectification: )  12.01.89 ...............
Date de décision portant sur modification: )

Ausgabe- und Veröffentlichungstag: )
Issue and publication date: )  10.05.89 .....................
Date d'edition et de publication: )

Patbl.Nr) 89/19
EPB no:) .........
Bull. no:)

Europäisches Patentamt

(19) European Patent Office

Office européen des brevets

(11) Numéro de publication: **0 113 626**
**B1**

(12) **FASCICULE DE BREVET EUROPEEN**

(45) Date de publication du fascicule du brevet: **26.10.88**

(51) Int. Cl.4: **C 12 N 9/96,** A 23 K 1/165

(21) Numéro de dépôt: **83402461.4**

(22) Date de dépôt: **19.12.83**

(54) **Procédé pour stabiliser des enzymes liquides, enzymes liquides ainsi stabilisées et aliment renfermant de telles enzymes notamment pour des animaux monogastriques.**

(30) Priorité: **20.12.82 FR 8221302**

(43) Date de publication de la demande:
**18.07.84 Bulletin 84/29**

(45) Mention de la délivrance du brevet:
**26.10.88 Bulletin 88/43**

(84) Etats contractants désignés:
**AT BE CH DE GB IT LI LU NL SE**

(56) Documents cités:
**FR-A-2 000 884**
**FR-A-2 084 752**
**FR-A-2 338 653**
**GB-A- 29 996**
**GB-A- 869 615**
**US-A-2 594 356**
**US-A-3 617 300**

**CHEMICAL ABSTRACTS, vol. 72, no. 1, 5 janvier 1970, page 192, no. 2154p, Columbus, Ohio, US**

(73) Titulaire: **SANDERS, Société Anonyme dite:**
**17 quai de l'Industrie**
**F-91200 Athis-Mons (FR)**

(72) Inventeur: **Assoumani, Bakri**
**D 2 - Les Millepertuis Avenue du Berry**
**F-91940 Les Ulis (FR)**

(74) Mandataire: **Lepeudry-Gautherat, Thérèse et al**
**ARMENGAUD JEUNE CABINET LEPEUDRY 6,**
**rue du Fg. St-Honoré**
**F-75008 Paris (FR)**

Courier Press, Leamington Spa, England.

# 0 113 626

**Description**

La présente invention est relative à un procédé pour stabiliser des enzymes liquides et aux enzymes stabilisées ainsi obtenues. De plus, elle concerne des aliments renfermant de telles enzymes stabilisées pour nourrir notamment des animaux monogastriques.

Les enzymes sont, actuellement de plus en plus utilisées industriellement, en particulier dans les procédés biotechnologiques faisant appel à des fermentations. De ce fait, on assiste depuis quelques années à la production d'enzymes de plus en plus actives à coût de plus en plus intéressant : cela a notamment pour conséquence que la quantité mise en oeuvre dans un même procédé diminue, ce qui a pour corrollaire un abaissement du coût de ce procédé.

En général, les enzymes sont commercialisées sous forme liquide ou de poudre, séchées ou coséchées avec différentes molécules de sucre afin d'augmenter leur stabilité thermique dans ce dernier cas. La plupart du temps, le séchage entraîne une perte d'activité de l'enzyme ce qui augmente par là-même le coût de l'activité enzymatique et par suite celui du procédé.

Sur le plan technologique, on utilise les enzymes principalement sous les deux formes suivantes : libres ou immobilisées selon différentes techniques. Dans le cas des enzymes immobilisées, on distingue habituellement les enzymes liées par adsorption, c'est-à-dire liaison covalente telle que par exemple la glucose isomérase produite et commercialisée par la société américaine CPC International Inc. et les enzymes incluses dans une capsule ou une matrice comme par exemple la glucose-isomérase produite et commercialisée par la société italienne Snam Progetti. Ainsi qu'on peut le remarquer, une enzyme peut être immobilisée des deux façons envisagées ci-dessus.

En règle générale, on réalisé la fixation d'enzymes lorsque le substrat est soluble ou dispersible : celui-ci peut, de ce fait, entrer directement en contact avec l'enzyme puisqu'aucun problème de barrière physique n'existe.

Dans le domaine alimentaire, tant humain qu'animal, plusieurs publications font état de l'utilisation de différentes enzymes pour pallier une déficience physiologique : ainsi on supplémentera une ration d'orge destinée à la volaille par de la-amylase ou de la β-glucanase, ou une ration d'ensilage de grain pour ruminants notamment par des protéases, de l'α-amylase ou des cellulase. Mais les résultats ne sont pas toujours concluants, c'est-à-dire que les enzymes ajoutées n'ont aucune action bénéfique et parfois même auraient un rôle négatif.

La quantité nécessaire d'enzymes liquides biologiquement efficace, s'avère souvent très faible : généralement inférieure à 150 ml pour 100 kg d'aliment fini. Ceci constitue un obstacle technologique à l'emploi des enzymes liquides au niveau de la production de l'aliment. En outre, les conditions pour conserver des enzymes liquides sont relativement contraignantes puisque notamment elles doivent être stockées à la température de +4°C.

Aussi un des buts de la présente invention est-il de fournir un procédé pour stabiliser des enzymes liquides qui permet de conserver celles-ci à la température ambiante.

Un autre but de l'invention est de fournir un procédé permettant d'obtenir une enzyme qui garde son activité pendant le stockage.

Un object de l'invention est une enzyme liquide stabilisée qui ne présente pour ainsi dire pas de perte d'activité lors des différentes phases de fabrication de l'aliment en comportant et ce quel que soit le type de l'aliment (farine, granulés ou miettes).

Un autre object de l'invention est un aliment contenant des enzymes stabilisées efficaces in vivo particulièrement destiné à l'alimentation des animaux monogastriques.

Ces buts et ces objets, ainsi que d'autres qui apparaîtront par la suite sont atteints par le procédé pour préparer des béta-glucanases ou des alpha-amylases liquides stabilisées conservant leur activité au stockage, caractérisé selon la présente invention, en ce qu'il consiste à pulvériser ces enzymes sur un support solide brassé en continu qui est un sous produit solide de la fermentation alcoolique des matières végétales, suivie éventuellement de distillation et présente une activité de l'eau inférieure à 0,55 et un pH voisin de 5.

De préférence, le support solide présente une granulométrie inférieure à 1 millimètre.

Avantageusement, la quantité d'enzymes est comprise entre 0,5 et 100 ml par kg de support solide.

De préférence, le support solide provient notamment de la fermentation alcoolique des grains de maïs ou de la production d'éthanol.

Ainsi qu'il a déjà été énoncé, la présente invention a également trait aux enzymes liquides stabilisées par un tel procédé ainsi qu'aux aliments en renfermant.

En pulvérisant une béta-glucanase ou une alpha-amylase liquide sur un support solide provenant de la fermentation alcoolique des grains de maïs, celle-ci est absorbée sur le support solide de telle sorte qu'elle peut être libérée en milieu aqueux pour hydroliser un substrat.

Les supports solides selon la présente invention sont déjà traités par des acides organiques afin d'éviter toute contamination.

Ainsi qu'il a été indiqué plus haut, ces supports solides constitués principalement par des sous-produits issus soit de la production d'éthanol comme les drêches épuisées de distillerie (distiller's spent grain ou DSG), soit de la fermentation alcoolique du maïs comme les fractions riches en protéines récupérées après la distillation. Ces fractions comprennent ce que l'on appelle des drêches de distillerie et

2

une fraction de solubles. Dans ce cas, les principaux sous-produits sont les drêches de distillerie de maïs contenant les solubles (corn distiller's dried grains with solubles ou CDGS), les drêches séchées de distillerie de maïs (corn distiller's dried grains ou CDG) et les solubles séchés de distillerie de maïs (corn distiller's dried solubles ou CDS). La valeur moyenne des principales caractéristiques physico-chimiques et la composition moyenne de ces différents produits sont regroupées dans le tableau I ci-après:

TABLEAU

| | Eau % | Mat. Azotées totales % | Mat. Gras % | Cellulose % | Mat. Min. % | Protéines vraies % | Activité de l'eau ($A_w$) | pH |
|---|---|---|---|---|---|---|---|---|
| C.D.G.S. | 9,2 | 27,3 | 7,6 | 7.3 | 5.9 | — | 0,50 | 4,46 |
| C.D.G. | 8 | 29,5 | 8 | 12,8 | — | — | | |
| D.S.G. | 6,8 | 27,5 | 9,8 | 7,5 | 4,5 | 17,5 | | |
| C.D.S. | 10 | 29,8 | — | 4,2 | — | — | | |

Tous les pourcentages sont en poids

Les principales caractéristiques des supports solides tels que ceux cités dans le tableau I, sont
— un pH relativement bas, de l'ordre de 5;
— une activité de l'eau (Aw) basse, inférieure à 0,55;
— une teneur en matière grasse faible afin d'éviter toute peroxydation de type enzymatique susceptible d'induire des radicaux libres pouvant dénaturer les enzymes liquides à stabiliser.

En outre, ces support solides ne doivent pas présenter des activités enzymatiques in-situ pouvant interférer avec celle des enzymes stabilisées.

La quantité d'enzymes liquides pulvérisée sur le support solide qui est brassé en continu, est de l'ordre de 0,5 à 100 ml par kg de support. En fait le taux maximal de pulvérisation est limité, d'une part, par l'activité de l'eau (Aw) maximale permettant la croissance des microorganismes — ce qui doit être évité — et, d'autre part, par la prise en masse du support et par là-même de la perte de fluidite. Le document FR—A—2 000 884 décrit un procédé pour concentrer des enzymes par absorption de solutions d'enzymes sur le son et séchage.

Or le son est un support contenant de 15 à 30% d'amidon hygroscopique et des sucres.

Lex exemples suivants ont pour rôle de permettre à l'homme du métier de réaliser la présente invention.

## Exemple 1

Dans un appareil de type "24 ACCELA-COTA DE MANESTY" utilisé pour enrober des comprimés, dont les orifices servant normalement à l'injection d'air chaud pour le séchage ont été bouchés, on introduit 6 kg de drêches séchées de distillerie de maïs (CDG). Tout en faisant tourner cet appareil à une vitesse d'environ 12 tours par minute, on pulvérise pendant 6 minutes une β-glucanase liquide au moyen d'un pistolet équipé d'une buse ayant un diamètre de 0,8 millimètre. Le débit de pulvérisation est de 50 ml/mn avec une pression d'air de $3,5.10^5$ Pascals. Après un temps d'homogénéisation de trois minutes environ, on arrête la rotation du tambour et on récupère la β-glucanase liquide stabilisée.

Cette enzyme ainsi stabilisée peut être ajoutée par exemple dans un aliment pour volaille dont la formule peut renfermer des taux d'orge compris entre 5 et 70% en poids. Le tableau II ci-dessous donne une formule de croissance pour volailles comportant une enzyme liquide stabilisée selon la présente invention.

| Constituants | % (en poids) |
|---|---|
| Maïs | 31,9 |
| Orge | 30 |
| Soja | 26,3 |
| Viande | 5 |
| Matière grasse | 4,3 |
| Composé minéral vitaminisé | 1,5 |
| Enzyme liquide stabilisée | 1 |

## Exemple 2

Dans un mélangeur horizontal à ruban de 5000 litres comportant 10 buses pour la pulvérisation, on introduit 2 200 kg de drêches de distillerie de maïs contenant les solubles (CDGS), la vitesse de rotation du mélangeur étant de 20 tours par minute. La pulvérisation d'un mélange de β-glucanase et d'α-amylase est réalisée pendant 6,5 minutes avec un débit de 140 l/h sous une pression d'air de $4,1.10^5$ Pascals.

Après 3 minutes d'homogénéisation, on recupère le mélange d'enzyme liquide stabilisée pouvant être introduit au taux de 0,5 ou de 1% en poids dans un aliment pour volailles pouvant contenir de 5 à 70% en poids d'orge.

Le procédé selon la présente invention conduit donc à la production d'enzymes liquides stabilisées sur un support solide, le tout constituant un prémix pouvant être utilisé pour fabriquer un aliment destiné notamment à des animaux monogastriques tels que des volailles.

**Revendications**

1. Procédé pour préparer des beta-glucanases ou des alpha amylases liquides stabilisées conservant leur activité au stockage, caractérisé en ce qu'il consiste à pulvériser ces enzymes sur un support solide brassé en continu qui est un sous-produit solide de la fermentation alcoolique de matières végétales suivie éventuellement de distillation et présente une activité de l'eau inférieure à 0,55 et a un pH de l'ordre de 5.

2. Procédé selon l'une quelconque des revendications 1 et 2, caractérisé en ce que ledit support solide présente une granulométrie inférieure à 1 millimètre.

3. Procédé selon l'une quelconque des revendications 1 à 3, caractérisé en ce que la quantité d'enzymes à pulvériser est comprise entre 0,5 et 100 ml par kg de support solide.

4. Procédé selon l'une quelconque des revendications 1 à 4, caractérisé en ce que le support solide est un sous-produit de la production d'éthanol.

5. Procédé selon la revendication 5, caractérisé en ce que le support solide est constitué de drêches épuisées de distillerie.

6. Procédé selon la revendication 5, caractérisé en ce que le support solide est constitué par un sous-produit de la fermentation alcoolique du maïs.

7. Enzymes liquides stabilisées par le procédé selon l'une quelconque des revendications 1 à 7.

8. Aliment, notamment pour animaux monogastriques, caractérisé en ce qu'il comprend de 0,5 à 1,5% en poids d'au moins une enzyme liquide stabilisée par le procédé selon l'une quelconque des revendications 1 à 7.

## Patentansprüche

1. Verfahren zum Herstellen stabilisierter, flüssiger β-Glucanasen oder α-Amylasen, die beim Lagern ihre Aktivität beibehalten, dadurch gekennzeichnet, daß diese Enzyme auf einen kontinuierlich gerührten festen Träger, welcher ein festes Nebenprodukt der gegebenfalls von einer Distillation gefolgten alkoholischen Gärung pflanzlicher Stoffe ist, aufgesprüht werden, sowie der feste Träger eine Wasseraktivität von unter 0,55 und einen pH-Wert in der Größenordnung von 5 aufweist.

2. Verfahren nach Anspruch 1, dadurch gekennzeichnet, daß der genannte feste Träger eine Teilchengröße von weniger als 1 mm aufweist.

3. Verfahren nach einem der Ansprüche 1 oder 2, dadurch gekennzeichnet, daß die Menge der aufzusprühenden Enzyme 0,5 bis 100 ml pro kg des festen Trägers beträgt.

4. Verfahren nach einem der Ansprüche 1 bis 3, dadurch gekennzeichnet, daß der feste Träger ein Nebenprodukt der Ethanolherstellung ist.

5. Verfahren nach Anspruch 4, dadurch gekennzeichnet, daß der feste Träger verbrauchter Treber aus der Destillation ist.

6. Verfahren nach Anspruch 4, dadurch gekennzeichnet, daß der feste Träger aus einem Nebenprodukt der alkoholischen Gärung von Mais besteht.

7. Stabilisierte, flüssige Enzym, hergestellt nach einem der Ansprüche 1 bis 6.

8. Nahrungsmittel, insbesondere für monogastrische Tiere, dadurch gekennzeichnet, daß es 0,5 bis 1,5 Gew.-% mindestens eines nach einem der Ansprüche 1 bis 6 stabilisierten, flüssigen Enzyms enthält.

## Claims

1. Process for preparing stabilised liquid beta-glucanases or alpha-amylases retaining their activity on storage, characterised in that it consists in spraying these enzymes onto a continuously stirred solid support, which is a solid by-product of the alcoholic fermentation, followed where appropriate by distillation, of vegetable matter, and possesses a water activity of less than 0.55 and has a pH of the order of 5.

2. Process according to Claim 1, characterised in that the said solid support possesses a particle size smaller than 1 millimetre.

3. Process according to either of Claims 1 to 2, characterised in that the quantity of enzymes to be sprayed is between 0.5 and 100 ml per kg of solid support.

4. Process according to any one of Claims 1 to 3, characterised in that the solid support is a by-product of ethanol production.

5. Process according to Claim 4, characterised in that the solid support consists of distiller's spent grain.

6. Process according to Claim 4, characterised in that the solid support consists of a by-product of the alcoholic fermentation of corn.

7. Liquid enzymes stabilised by the process according to any one of Claims 1 to 6.

8. Feed, especially for monogastric animals, characterised in that it comprises from 0.5 to 1.5% by weight.